# EUROPEAN PATENT APPLICATION

(11) **EP 4 331 542 A1**
(43) Date of publication of application: **06.03.2024**
(21) Application number: 22193248.6
(22) Date of filing: 31.08.2022
(51) Int. Cl.: A61F 9/008

(54) **APPARATUS FOR PROVIDING RETINAL THERAPY, METHOD FOR TRACKING A CHANGE IN RETINAL CELLS' FUNCTIONAL MODIFICATION**

(71) Applicant: Oculox Technologies SA, 6933 Muzzano (CH)
(72) Inventor: PIFFARETTI, Filippo, 6512 Giubiasco (CH); FAURE, Pascal, 1025 Saint Sulpice (CH)
(74) Representative: P&TS SA (AG, Ltd.)

(57) **Abstract**

The invention concerns an apparatus for providing retinal therapy of an eye comprising:
- a first light source configured to emit a treatment beam with a first wavelength to a selected area of the retina, whereby the retina is external to the apparatus;
- a retinal functional tracking means comprising a second light source and a detector, wherein the second light source is arranged to emit light within a spectrum to the selected area of the retina, and wherein the detector is configured to analyse reflected light by the retina at the selected area, wherein the detector is adapted to determine a change in the spectrum of the reflected light; and wherein
- the apparatus is configured to adapt a therapeutic dose emitted by the first light source in response to the determination of a change in the spectrum of the reflected light.

The invention concerns also a method for detecting a change in retinal cells of an eye stimulated by a treatment beam of a first light.

## Description

### Technical domain

The present invention concerns an apparatus suitable for providing retinal therapy and a method suitable for tracking a change in retinal cells' functional modification. The disclosure further concerns a confocal retinal scanning module, which comprises the apparatus.

The apparatus and the corresponding method enable physicians to provide a treatment of the retina comprised in an eye while optimising the therapeutic dose and reducing or avoiding the risks of overtreatment of the retina.

### Related art

For many years, sub-threshold retina treatments, e.g., Sub-threshold Diode Micropulse laser therapy, have been widely investigated and applied for the treatment of retinal disorders.

The state-of-the-art technique for retina laser treatment is the so-called "Selective Retina Therapy", which presents an exciting efficacy profile in a very narrow power range, close to the neuroretina damage threshold.

However, an exact automatic feedback system is required for laser treatments safely to avoid neuroretina cellular damages. A deviation of the tissue heat transfer characteristics or a small procedure error can cause overtreatment. In return, the delicate neuroretina is irremediably affected by excessive thermal stresses.

The principle of action for this therapy or treatment stems from a deadly laser insult electively delivered to the cells of the Retinal Pigmented Epithelium (RPE).
The laser pulse length is limited to a fraction of a few microseconds. This may ensure selective heat confinement within some micrometres from the primary absorption chromophore (RPE-melanin).

Beam irradiance is normally selected to favour a specific laser-tissue interaction, e.g. creating the necessary RPE cells damages or stress to induce necrosis/apoptosis while avoiding the heat waves being dispersed to adjacent more delicate cells.

Selective Retina Therapy favours the photomechanical interaction induced by the important heat gradient generating microbubbles (vaporization and cavitation effects) close to the RPE-melanin gains.

The methods as herein outlined before can be hazardous, as they work on the borderline to the threshold deposited energies above which irreversible scotoma is induced.

Other laser treatment modalities are widely investigated or used clinically, but all these result from a historical regression.

The clinical community was firmly convinced that a morphological tissue modification was a sine qua non-condition to trigger the expected healing effect. This belief, the necessity to devitalize deficient cells to hope for the replacement, is still very rooted in the clinical community.

It is thus an objective of the present invention to provide an apparatus for providing retinal therapy and a method for detecting a change in retinal cells, eliminating at least some of the disadvantages of devices and corresponding methods known from the prior art.

It needs to be noted that the apparatus and the method as suggested is suitable for use in the therapy of retinal disorders. However, no treatment or diagnosis is indicated by the present disclosure.

### Short disclosure of the invention

The invention concerns an apparatus for providing retinal therapy of an eye comprising:
- a first light source configured to emit a treatment beam with a first wavelength to a selected area of the retina, whereby the retina is external to the apparatus;
- a retinal functional tracking means comprising a second light source and a detector, wherein the second light source is arranged to emit light within a spectrum to the selected area of the retina, and wherein the detector is configured to analyse reflected light by the retina at the selected area, wherein the detector is adapted to determine a change in the spectrum of the reflected light; and wherein
- the apparatus is configured to adapt a therapeutic dose emitted by the first light source in response to the determination of a change in the spectrum of the reflected light.

The eye can be an organ of vision of a human or an animal. The eye can be external to the apparatus, preferably without being in contact with the apparatus as such.

The first light source is also known as "treatment light source". The first light source can be configured to emit a therapeutic dose of light which can be comprised in the treatment beam, whereby the term "therapeutic dose" means the emission of a dose of light, such as a treatment light pulse, that is required to elicit a desired therapeutic response in the posterior segment of the eye in the treatment of disease or ailment.

The first light source can be any sort of light source that is suitable to emit a therapeutic dose or treatment beam with a specific wavelength. A broadband light source, such as a xenon lamp in combination with an optical filter, for allowing one specific wavelength only can be used as a light source. Preferably, a laser diode can provide the first light source, emitting only within a specific wavelength range.

The selected area can be any part of the retina. It can comprise a three-dimensional space (voxel) comprised of one specific layer of the retina, such as the pigmented layer or the inner nuclear layer, but also a three-dimensional space composed of multiple retinal layers in which the therapeutic light dose penetrates. Preferably the selected area of the retina can be the retinal pigment epithelium.

Alternatively, the selected area can comprise individual cells contained in one layer of the retina only. The said cells can be targeted by the first light source individually if the wavelength is smaller than the volumetric extension of the cells.

The second light source can comprise a broadband light source, as it can also be used for the first light source in combination with an optical filter to allow only light within a specific spectrum of wavelengths to pass. The light emitted by the second light source or by the apparatus can also be in the form of a beam.

The light emitted by the second light source may not result in a therapeutic effect and thus has lower irradiance and radiant energy when compared to the first light source.

The apparatus and/or the first and second light sources can be configured to emit light (or beams) simultaneously. It means that the first light source can emit a treatment beam, and the second light source can emit light (or a beam) within a spectrum at the same time. Alternatively, the apparatus and/or the first and second light sources can be configured to operate asynchronously, meaning that only one source emits light (or a beam) at a specific time.

The area targeted by the light (or beam) emitted by the second light source and reflected by the retina can be the same as the area targeted by the treatment beam provided by the first light source.

The detector can be provided by a light-sensitive diode sensitive to the spectrum of light emitted by the second light source and/or light reflected by the retina.

A further means can be provided with the detector suitable to fractionize the spectrum of the received light into several wavelengths. Preferably the detector can be provided by a photo sensor in the form of an Avalanche-Photodiode-Array in combination with an optical grating to extract spectral data from the reflected light.

The detector can be adapted to determine a change in the spectrum of the reflected light. This can comprise the detection of a change in the amplitude of light in a specific wavelength or a shift in the spectrum, such as a redshift or blueshift, corresponding to an increase or decrease in the wavelengths contained in the spectrum of the reflected light.

The apparatus can be configured to adapt to the therapeutic dose emitted by the first light source. Adjusting the therapeutic dose can increase or decrease the fluence at the selected area of the retina.

The therapeutic dose can be adapted in response to the determination of a change in the spectrum of the reflected light.

A change in the spectrum may be caused by the stimulated photochemical reactions springing from the absorption of the treatment beam by the targeted cell's chromophore .

However, this is not the only known mechanism that may cause a change in the spectrum. Other mechanisms are but are not limited to molecular, cellular, and tissue avalanche biochemical reactions.

Since the treatment beam is emitted by the first light source at a specific wavelength and the therapeutic dose is varied, the general tissue interaction (photochemical and photothermal) can be precisely stimulated and controlled by the apparatus, which is an advantage over devices and methods known from the prior art.

The spectrum of the light emitted by the second light source can include light in a wavelength between 700nm and 1400nm, more preferably between 700nm and 1000nm, and most preferably between 700nm and 900nm. Light with wavelengths in this spectrum is also referred to as near-infrared light. Light within the same spectrum can be comprised in the beam for determining or detecting a change in the retinal cells.

The light emitted by the first light source can be light of a specific wavelength selected from a range from 775nm to 837nm, preferably from 800nm to 820nm and most preferably from 808nm to 812nm. Preferably, but not exclusively, the wavelength of the light emitted by the first light source can account for 810nm.

Alternatively, the light emitted by the first light source can be light of a specific wavelength selected from a range from 785nm to 845nm, preferably from 805nm to 840nm and most preferably from 825nm to 835nm. Preferably, but not exclusively, the wavelength of the light emitted by the first light source can account for 830nm.

Light within the same specific wavelength can be comprised in the treatment beam.

In one embodiment of the apparatus, according to the invention, the therapeutic dose can be characterized by at least one parameter of the first light source, including radiant power, irradiance, exposure duration, repetition intervals, and fluence or a combination thereof.

In another embodiment of the apparatus, according to the invention, the first light source can be configured to emit a plurality of consecutive light pulses within an exposure duration, wherein each light pulse can be characterized by an amplitude, a pulse period, and a pulse width. The exposure duration can correspond to the time used to treat the selected area of the retina.

The therapeutic dose can be characterized by the peak radiant power, pulse width, the number of consecutive pulses within the exposure duration, and the fluence or a combination thereof.

The pulse width of the pulses or the therapeutic dose can be less than one-half of a pulse period, preferably less than one-third of a pulse period.
Alternatively, or in addition, the pulse period can be equal to or less than 500 µs. Preferably, equal to or less than 250 µs.
Alternatively or in addition, the exposure duration can be equal to or less than 500 ms. Preferably equal to or less than 250 ms.

The values as provided hereinbefore can prevent overtreatment.

In a further embodiment of the apparatus, according to the invention, the change in the spectrum of the reflected light can be caused by the treatment of retinal cells. The change in the spectrum can be detected while energizing the first light source for applying a therapeutic dose.

This is advantageous as the effect on retinal cells can be observed and detected while the therapeutic dose is applied, particularly while the first light source is energized.

In another embodiment of the apparatus, according to the invention, the apparatus can comprise a control device.

The control device can be connected to the first light source and the retinal functional tracking means. The control device can be configured to control, at least one of the parameters of the first light source emitting the treatment beam in response to the determination of a change in the spectrum of the reflected light.
By controlling the first light source, the treatment beam or the therapeutic dose is varied accordingly.

For example, the control device can be an electronic control device such as a microcontroller, FPGA, or a combination thereof.

The control device may feature analog and/or digital inputs and outputs and can be configured to receive analog or digital values from the detector. The control device may also be arranged to receive or send control commands from or to the first light source to control the parameter of the therapeutic dose as set out hereinbefore.

However, the functionality of the control device is not limited to the features as explained before. The control device may also set or control the operational parameters of the second light source.

The control device can be further adapted to control or set the at least one of the parameters of the first light source while the first light source emits a therapeutic dose to the retina. Alternatively, or in addition, the control device can be adapted to control or set the at least one parameter of the first light source after the emission of a therapeutic dose. This means that the emission can be paused between two consecutive treatments.

The control device can be configured to control the parameter of the first light source related to at least one treatment objective. At least one treatment objective can be characterized by the thermal, biochemical and systemic, and collective cellular activation of retinal cells or a combination thereof.

The treatment objective can be set via an input device, such as a human-machine interface, prior to the treatment. The control device automatically adapts the dose parameter in accordance with the set treatment objectives and in response to the detection of a change in the spectrum of the reflected light.

This ensures advantageously that a physician can set the treatment objective without any further intervention during the treatment using the apparatus. The treatment of the retina is performed in an automated or semi-automated way, at least over the timespan of an exposure duration.

The control device can be configured to control the power of the first light source between a minimum and a maximum value corresponding to the at least one treatment objective. The power in return can correspond to total dose delivered to the tissue (beam fluence). The minimum and a maximum value can be different for each of the at least one treatment objective.

In a further embodiment of the apparatus, according to the invention, the apparatus can comprise a lens configured to concentrate the light emitted by the first light source and the second light source. The light of the said light sources can use the lens as a common optical path between the light sources and the eye.

The need for further units of the apparatus, for instance, a second lens, can be omitted by using a common optical path shared by the said light sources. This reduced complexity and costs, which is desirable.

In a different embodiment of the apparatus, according to the invention, the retinal functional tracking means can comprise a wavelength dispersive element, such as a dispersive prism, an optical grating, suitable to extract spectra of the reflected light by the retina at the selected area.

In a further embodiment of the apparatus, according to the invention, the first wavelength (e.g. 830 nm) can be excluded from the determination by retinal functional tracking means.

This can be useful when the treatment beam is emitted and the spectrum of the reflected light is determined simultaneously. Interferences or superpositions can be avoided which may improve the sensitivity for detecting changes in the spectrum of the reflected light. The use of notch filters for filtering interferences or superpositions can be omitted, which leads to a simplification of the apparatus.

In another embodiment of the apparatus, according to the invention, the retinal functional tracking means can be configurated as an optical coherence tomograph or a confocal scanning microscope.

The invention also concerns a method for detecting a change in retinal cells' functional modification of an eye which can be stimulated by a treatment beam of a first light source, comprising the steps of:
- Emitting a treatment beam with a first wavelength to a selected area of a retina;
- Emitting a functional excitation beam to the selected area of the retina, wherein the measurement beam includes light within a spectrum;
- Receiving reflected light by the retina within the spectrum;
- Determining a change in the spectrum of the reflected light.

The change in the light spectrum can be caused by an activation of mitochondrial respiration activities of retinal cells. However, other changes in the retinal cells can also cause a change in the spectrum of the reflected light. These different causes can be considered or disregarded depending on the treatment objective.

The method is advantageous, as the response of the cells corresponding to the stimulation by the therapeutical dose can be determined and/or observed.

The method can be performed by or on the apparatus as set out in at least one of the embodiments disclosed hereinbefore.

In one embodiment of the method, according to the invention, the method can comprise the step of adapting a parameter of a therapeutic dose in response to the determination of a change in the spectrum of the reflected light.

In another embodiment of the method, according to the invention, the treatment beam and the functional excitation beam can be emitted simultaneously.

In a further embodiment of the method, according to the invention, the step of determining a change in the spectrum of the reflected light can be performed while emitting the treatment beam and/or applying the therapeutic dose.

This step can be advantageous as the influence of the treatment beam and/or the therapeutic dose on the retinal cells can be observed or detected while the treatment beam and/or the therapeutic dose is applied to the retinal cells. This can prevent overtreatment.

Alternatively, the step of determining a change in the spectrum of the reflected light can be performed after emitting a therapeutic dose.

This step can also be advantageous as the result of the therapeutic dose on the retinal cells can be observed or detected after the therapeutic dose was applied to the retinal cells.

Preferably the step of determining a change in the spectrum of the reflected light can be performed while and after emitting a treatment beam and/or a therapeutic dose to retinal cells.

The invention finally concerns a confocal retinal scanning module, comprising the apparatus set out in at least one of the embodiments disclosed hereinbefore.

The apparatus comprised by the confocal retinal scanning module can perform the method set out in at least one of the embodiments disclosed hereinbefore.

Confocal retinal scanning modules, known from the prior art, can be combined or refurbished using the apparatus as disclosed.

Physicians can provide treatment with a device that they know well. In addition, can they visualize if the treatment provided by the apparatus was successful, as physicians may notice visible changes in the retinal cells. Effective and lasting functional changes reverts pathological structural changes characteristics of chronic degenerative pathologies.

Even though the embodiments or aspects as disclosed herein before might discuss different aspects of the invention, they may be combined individually or form a combination thereof in an apparatus or a confocal retinal scanning module when technically feasible and useful. However, they might also be considered separately when necessary.

### Short description of the drawings

Exemplar embodiments of the invention are disclosed in the description and illustrated by the drawings in which:
**Fig. 1a** illustrates an apparatus according to the invention schematically.
**Fig. 1b** illustrates the tissue stimulation by the treatment beam and the corresponding reaction of the cells.
**Fig. 2** illustrates an optical treatment and functional tracking optical module of the apparatus according to the invention schematically.
**Fig. 3** shows a total therapeutic dose in the form of a repetition of light pulses schematically.
**Fig. 4** illustrates an optical power module of the apparatus according to the invention schematically.
**Fig. 5** shows a possible configuration of the optical treatment unit as of **Fig. 2** schematically.
**Fig. 6** illustrates a possible configuration of the optical functional tracking unit as of **Fig. 4** schematically.

### Examples of embodiments of the present invention

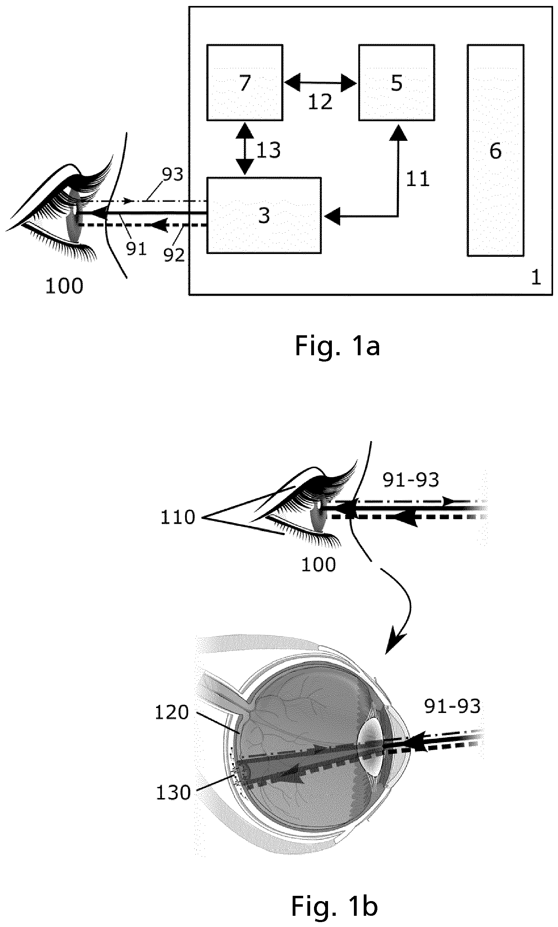

Concerning **Fig. 1a****,** an example of an apparatus 1 according to the invention is illustrated. The apparatus can be a part of or integrated into a fundus camera or another optical device typically used by physicians. However, the apparatus can also be configured as an independent, standalone device.

The apparatus 1 consists, in this example, of an optical treatment and functional tracking module 3, an optical power module 5, and a control module 7. The modules 3, 5, 7 can be placed in one common housing.

A front lens (not illustrated) of the apparatus 1 is directed towards an eye 100, whereby the optical treatment and functional tracking module 3 emit a treatment beam 91 and a functional excitation beam 92 towards the eye 100. The said lights pass through the front lens before entering the eye 100.

A portion of the functional excitation beam 92 is reflected by the retina comprised in the eye 100 and enters through the front lens of the optical treatment and functional tracking module 3 as reflected light by the retina 93. The same applies to the treatment beam 91. Some portions of the treatment beam 91 may be reflected by the by the retina 93 and may enter the of the optical treatment and functional tracking module 3 as reflected light.

The optical treatment and functional tracking module 3 determines the spectrum of the reflected light by the retina 93 and the apparatus adapts some parameters, such as the pulse width of the light pulse comprised in the treatment beam 91 in response to the detection of a change in the spectrum of the reflected light by the retina 93.

The optical power module 5 supplies the optical treatment and functional tracking module 3 with the necessary optical power to emit a treatment beam 91 to the eye 100. The optical power is transmitted from the optical power module 5 to the optical treatment and functional tracking module 3 through an optical power and communication bus 11, whereby the said bus 11 also is used for communication of operational and/or control commands parameters between the optical power module 5 and the optical treatment and functional tracking module 3.

The optical portion of the optical power and communication bus 11 is configured with an optical fiber that is suitable to conduct light without high losses.

The operation of the optical power module 5, the optical treatment and functional tracking module 3 is controlled by the control module 7. The control module 7 is communicatively connected to the said modules through communication buses 12, 13.

The control module 7 is also destined to adapt and control the emission of the treatment beam 91 in response to a change in the spectrum detected by the optical treatment and functional tracking module 3. The control module 7 directly influences the light generation in the optical power module 5.

The control module 7 can be perceived as an overarching control device, configurated to control the operation of the apparatus 1 and providing (technical) diagnostic features for all other modules 3, 5.

The apparatus 1 further comprises a human-machine interface (HMI) 6 in form of a touchscreen, touch pad and/or a keyboard. The HMI 6 is used, for instance, by an ophthalmologist to enter treatment parameters, such as treatment objectives, pupil diameters, etc.

A footswitch, as a part of the HMI 6 is used by the physician to activate the emission of the treatment beam 91 and the functional excitation beam 92.

The functionality of the modules 3, 5, 7 as herein disclosed before, is exemplified. Some of the functionalities can be combined in one module or the apparatus 1 may comprise further modules to deconcentrate the functionality further.

In a highly simplified description of the present apparatus 1 an optical-electrical feedback control is provided by the modules, in which the optical treatment unit 31, in combination with the retinal cells correspond to the system to be controlled, in which the optical functional tracking unit 32 corresponds to the sensor and in which the control module 7 corresponds to the controller.

Fig. 1b illustrates an example of the tissue stimulation by the treatment beam and the corresponding reaction of the cells.

The treatment beam 91 and the functional excitation beam 92 are emitted in the direction of the eye 100. They enter the pupil of the eye 100 as the envelope 110 does not cover the cornea. The beams 91, 92 penetrate the eyeball and impinge on the retina 120. Portions of the beam/light of the functional excitation beam 92 are reflected by the retina 120 and reverberate in the direction of the pupil as reflected light 93. Portions of the treatment beam 91 may also be reflected by the retina 120. The reflected light 93 leaves the eyeball and enters the apparatus 1 as shown in Fig. 1a.

The treatment beam 91 causes a tissue stimulation 130 in the related cells. Different cells or layers of the retina may be targeted by the treatment beam 91. In reverse, the effect of the tissue stimulation 130 causes a change in the spectrum of the reflected light 93.

It needs to be noted that depending on the stimulation (intensity, duration, etc.) the spectrum may change differently. For example, a stimulation with high intensity may cause a redshift in the spectrum of the reflected light 93.

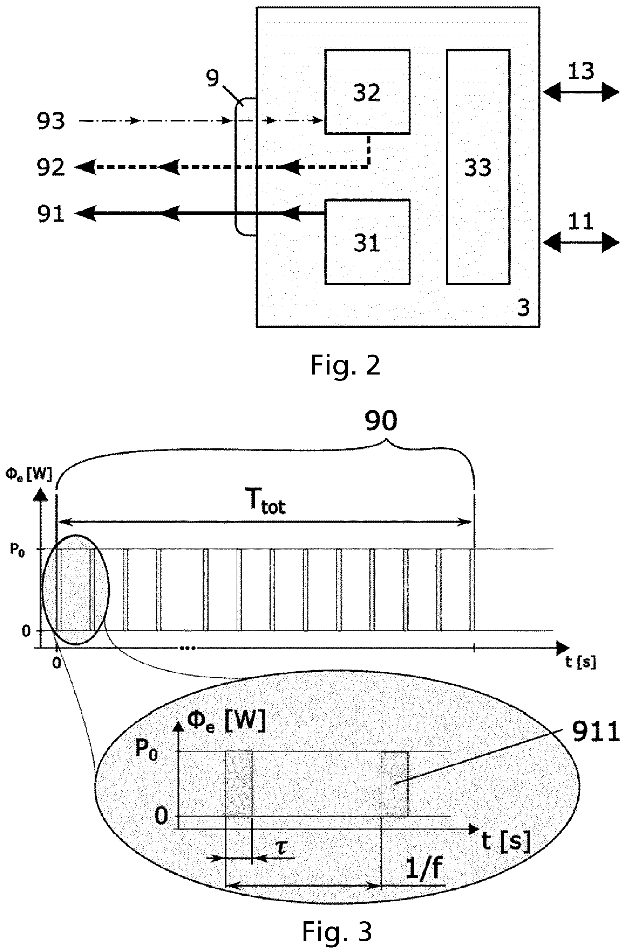

Fig. 2 illustrates an example of an optical treatment and functional tracking module 3 of the apparatus according to the invention.

The optical treatment and functional tracking module 3 comprises a front lens module 9, an optical treatment unit 31, optical functional tracking unit 32, and a laser beam exit module 33.

The optical treatment and functional tracking module 3 further comprises input and output ports arranged so the module can be connected to the communication bus 13 and the optical power and communication bus 11.

The optical treatment unit 31 also comprises a plurality of optical elements, such as mirrors, lens arrays and other elements for filtering light. The said optical elements are used to guide, divert, focus and/or concentrate in combination with the front lens module 9 treatment beam 91 on selected areas of the retina.

The phrase "in combination with" means in this respect that the optical path provided for the treatment beam 91 is a combination of the optical elements comprised in the optical treatment unit 31 and the front lens module 9.

The light that is focused or concentrated by the optical treatment unit 31 is provided by the laser beam exit module 33 and originates from the optical power module 5. The light emanating from the optical power module 5 is provided over the optical power and communication bus 11.

The optical functional tracking unit 32 also comprises a plurality of optical elements, such as mirrors, lens arrays and additional elements for filtering light. In addition, the optical functional tracking unit 32 includes a light source capable of providing light within a specific spectrum.

The light source used in this example for the optical functional tracking unit 32 is a near-infrared light source with wavelengths ranging between 750 nm and 980 nm.

The near-infrared light source emits the light passing through the plurality of optical elements and the front lens module 9 as functional excitation beam 92 into the direction of the eye. The retina of the eye reflects the functional excitation beam 92, whereby the light returns in the form of reflected light by the retina 93 and passes through the front lens module 9 and the plurality of optical elements comprised in the optical functional tracking unit 32.

A detector in the form of an Avalanche-Photodiode-Array (APA) and an optical grating placed in front of the sensor surface of the APA. The detector is used to extract spectral data from the reflected light by the retina and detects changes in the spectrum of the reflected light by the retina 93, while the treatment beam 91 is applied to the retina.

The treatment beam 91, the functional excitation beam 92 and the reflected light by the retina 93 share the same optical path as they pass at least through the front lens module 9 altogether.

Fig. 3 illustrates an example of a total therapeutic dose 90 in the form of a repetition of treatment light pulses 911.

Light is emitted via the front lens module to the eye in the form of such a therapeutic dose 90. One therapeutic dose 90 applied to the retina can comprise in this example a plurality of periodic treatment light pulses 911. The total therapeutic dose 90 is delivered to the targeted tissues within a certain period of time, the total exposure duration Tₜₒₜ.

The treatment beam 91 delivers the total therapeutic dose 90 to the targeted tissues with a pulsed protocol. The total exposure duration *T*ₜₒₜ is split into multiple pulse periods 1/f in which a treatment light pulse 911 is emitted. As indicated on the abscissa of the graph, the treatment light pulse 911 in each pulse period 1/f has a specific width corresponding to the pulse width τ.

Typical values used in this example are a pulse period *1*/*f* of 400µs, a pulse width τ of 100µs, which correspond to a duty cycle of 25%, and an exposure duration Tₜₒₜ of 400ms. These values result in the transmission of 1000 pulses for treatment of a selected area on the retina.

Each light pulse is characterized by a peak power amplitude *P*₀, as indicated on the ordinate of the graph. In this example accounts for the peak power amplitude P₀ to 40 W. The peak power amplitude P₀ may correspond to the radiant flux Φₑ emitted by the apparatus 1.

The total therapeutic dose 90, expressed in terms of, "irradiance" x "total exposure duration", or fluence applied to the retina can be controlled by the amplitude of the radiated pulse P₀, the variation of total exposure duration Tₜₒₜ, and/or the pulse width τ.

As indicated before, the total therapeutic dose 90 is adjusted in response to detecting a change in the spectrum of the reflected light by the retina.

Therefore, the values as provided before are only applicable for a stable operational point and might be subject to change during the application of the therapeutic dose 90.

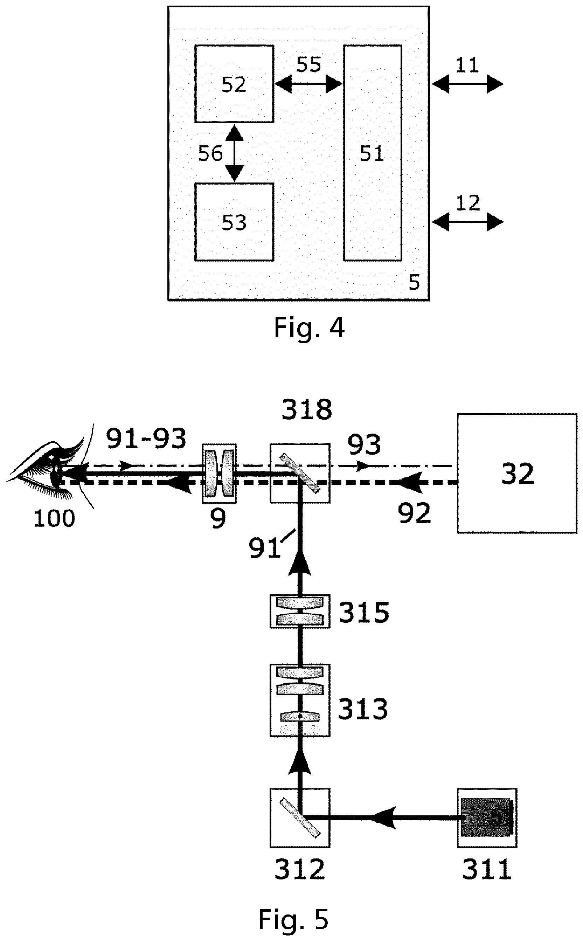

Regarding Fig. 4, an example of an optical power module 5 of the apparatus according to the invention is illustrated.

The optical power module 5 comprises a laser interface module 51, a laser control module 52 and a laser beam output module 53.

The optical power module 5 also comprises input and output ports such that the said module can be connected to the communication bus 12, and to the optical power and communication bus 11.

The laser interface module 51 comprises a power supply unit for supplying the laser control module 52 with electrical energy through the communication and electrical power supply bus 55.

The laser interface module 51 comprises a switching unit configured to interrupt or permit the supply of electrical energy to the laser control module 52. The switching unit is coupled to the footswitch of the HMI. The flow of electrical energy to the laser control module 52 is permitted if a physician activates the footswitch.

The laser control module 52 includes a light source in the form of a laser diode. The laser diode is configured to emit light with a specific wavelength, preferably with a wavelength of 810 nm or 830 nm. Due to tolerances in the manufacturing and/or during operation of the laser diode the wavelength might have a tolerance of +/- 5nm.

The laser diode is supplied with electrical energy provided by the laser interface module 51. The electrical energy is varied by a control device in the laser control module 52. The said control device controls or sets the energy that is supplied to the laser diode in a way that a pulse pattern is generated, as discussed in regards to Fig 3 hereinbefore.

The said control device can also be connected through the communication bus 13 to the control module 7 of Fig. 1a. The control device receives control commands or settings from the control module 7 of Fig. 1a to set the pulse pattern and finally the therapeutic dose 90 in response to the detection of a change in the spectrum, which was detected by the optical functional tracking unit 32 of Fig. 2 and corresponding to a treatment objective that a physician set via the HMI of Fig. 1a.

However, this might not be the only possibility. The said control device might also be directly connected to the optical functional tracking unit 32 of Fig. 2, and receive commands to adapt the treatment beam 91 directly in response to detecting a change in the spectrum.

This may reduce the latency of the communication buses 12, 13 and improve the reactivity of adapting the total therapeutic dose 90.

The light which is emitted by the laser diode is transmitted to the laser beam output module 53. The laser beam output module 53 connects the light optically with the optical power and communication bus 11. The optical fiber comprised in the optical power and communication bus 11 conducts the light to the laser light input module of Fig. 2.

Fig. 5 shows an example configuration of the optical treatment unit as of Fig. 2.

In the first optical path, a treatment light source 311 generates with the use of a laser diode a treatment beam 91, having a wavelength of 830 nm.

The treatment beam 91 is redirected by an optical mirror 320 and enters the tissue layer selection 313 lens array. The said lens array 313 comprises multiple lenses, whereby at least one lens can vary its position to change the focal point (focus length) corresponding to the tissue layer which needs to be targeted.

The treatment beam 91 subsequently enters a second lens array 315 to further concentrate the treatment beam 91. Finally the treatment beam 91 arrives at the dichroic mirror 318 where it is redirected toward the front lens module 9.

The treatment light source 311, the optical mirror 312, and the lens arrays 313, 315 can be components of the optical treatment unit 31 as illustrated in **Fig. 2****.**

The treatment beam 91 exits the front lens module 9 and is emitted into the direction of the eye 100.

In a second optical path, the optical functional tracking module 32 is energised at the same time, as the treatment light source 311 and thus emits a functional excitation beam 92 into the direction of the dichroic mirror 318. The dichroic mirror 318 directs the functional excitation beam 92 towards the front lens module 9 and exits the front lens module in the direction of the eye 100.

Portions of the functional excitation beam 92 are reflected by the retina comprised in the eye 100 and enter as reflected light by the retina 93 the front lens module 9 into the direction of the dichroic mirror 318. The reflected light by the retina 93 travels through the dichroic mirror 318 and uses the same optical path as the functional excitation beam 92.

The reflected light by the retina 93 enters the optical functional tracking unit 32, where the light i.a. is decomposed into its spectral components. The remaining functionalities are in line as explained in connection with **Fig. 2****.**

The third optical path thus ranges between the front lens module 9 and the eye 100 and is shared by the treatment and functional excitation beam 91, 92 and the reflected light from the retina 93.

It can be noticed that the dichroic mirror 318 functions as an optical collector and deflector point, where multiple optical paths meet.

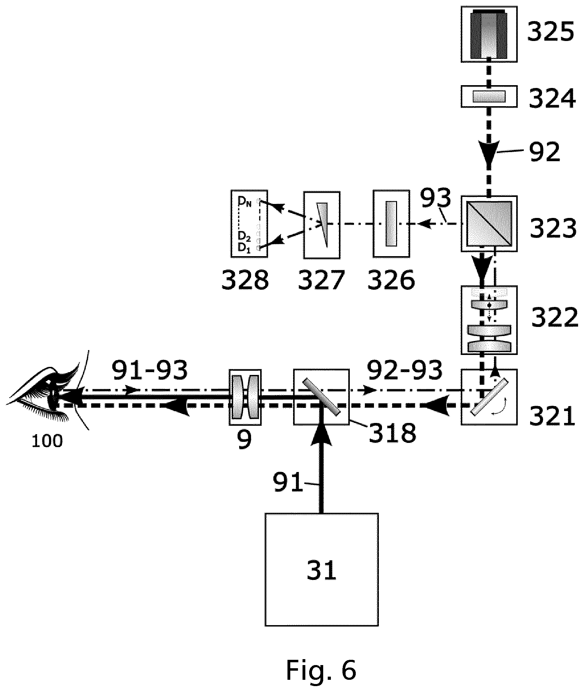

**Fig. 6** illustrates a possible configuration of the optical functional tracking unit as of **Fig. 4****.**

The treatment beam 91 in the first optical path is generated by the optical treatment unit 31. The optical treatment unit 31 can comprise the components as illustrated in Fig. 5. The treatment beam 91 is redirected at the dichroic mirror 318 into the direction of the front lens module 9. The treatment beam 91 exits the front lens module 9 and is emitted into the direction of the eye 100.

In a second optical path, light is generated by the broadband near infrared (NIR) light source and concentrated and/or filtered by the excitation pinhole 324 to emit the functional excitation beam 92.

The functional excitation beam 92 passes through the excitation/emission cube 323 and travels towards the tomogram selection optics 322. The tomogram selection optics 322 comprises multiple lenses, whereby at least one lens can vary its position to change the focal point (focus length) on the retina. The focal point on the retina for the functional excitation beam 92 can be the same focal point as for the treatment beam 91.

Upon leaving the tomogram selection optics 322, the functional excitation beam 92 is redirected by the 2-D scanning system 321 comprising a movable mirror. The position of the excitation beam 92 on the retina can be variably selected by changing the position of the mirror. The position or selected area on the retina of the functional excitation beam 92 and the treatment beam 91 can be exactly the same.

It is also possible that the functional excitation beam 92 targets a different position or area, which can be useful to monitor the tissue interaction of the treatment beam 91 in the environmental tissue or different layer of the retina.

The excitation beam 92 travels from the 2-D scanning system 321 to the dichroic mirror 318, whereby the dichroic mirror 318 directs the functional excitation beam 92 towards the front lens module 9 and exits the front lens module into the direction of the eye 100.

The reflected light by the retina 93 travels back through the front lens module 9, the dichroic mirror 318, the 2-D scanning system 321, the tomogram selection optics 322 and finally enters the excitation / emission cube 323, where it is redirection towards the notch filter and emission pinhole 326.

The notch filter and emission pinhole 326 can be used for stray light exclusion.

An optical dispersive grating 327 is placed in front of the line detector 328. The optical dispersive grating 327 is used to split the reflected light by the retina 93 into different wavelengths, whereby the line detector 328 captures the light level of the different wavelengths.

In conclusion, the apparatus can detect a change in the spectrum of the reflected light by the retina 93, preferably at selectable positions or areas on the retina, thanks to the 2-D scanning system.

The arrows as illustrated in all figures indicate in which direction the light travels and/or the beam is emitted or reflected.

All example embodiments present a limited selection of possible configurations of the apparatus and/or the confocal retinal scanning module comprising the apparatus. However, different other configurations can solve the technical problem as set out in the initial section of this disclosure.

### Reference signs

- 1: apparatus
- 3: optical treatment and functional tracking module
- 5: optical power module
- 6: human machine interface (HMI)
- 7: control module
- 9: front lens module
- 11: optical power and communication bus
- 12, 13: communication bus
- 31: optical treatment unit
- 32: optical functional tracking unit
- 33: laser beam exit module
- 51: laser interface module
- 52: laser control module
- 53: laser beam output module
- 55: communication or control bus
- 56: laser light bus
- 90: (total) therapeutic dose - integral on light pulse envelope
- 91: treatment beam
- 92: functional excitation beam
- 93: reflected light by the retina while stimulated by excitation beam
- 100: eye
- 110: envelope
- 120: retina
- 130: tissue stimulation
- 311: treatment light source
- 312: optical mirror
- 313: tissue layer selection - treatment
- 315: relay lenses
- 318: dichroic mirror
- 321: 2-D scanning system
- 322: tomogram selection optics
- 323: excitation / emission cube
- 324: excitation pinhole
- 325: broadband NIR light source
- 326: notch filter (stray light exclusion) and emission pinhole
- 327: optical dispersive grating
- 328: line detector
- 911: treatment light pulse
- *P*₀: peak power amplitude of the radiated pulse
- 1/f: pulse period
- Tₜₒₜ: exposure duration
- Φₑ: radiant flux/power
- τ: pulse width.

## Claims

1. An apparatus (1) for providing retinal therapy of an eye (100) comprising:
- a first light source (31) configured to emit a treatment beam (91) with a first wavelength to a selected area of the retina (120), whereby the retina (120) is external to the apparatus;
- a retinal functional tracking means (32) comprising a second light source (325) and a detector (328), wherein the second light source (325) is arranged to emit light (92) within a spectrum to the selected area of the retina (120), and wherein the detector (328) is configured to analyse reflected light by the retina (93) at the selected area, wherein the detector (32) is adapted to determine a change in the spectrum of the reflected light (93); and wherein
- the apparatus (1) is configured to adapt a therapeutic dose (90) emitted by the first light source (31) in response to the determination of a change in the spectrum of the reflected light (93).

2. The apparatus (1) of the preceding claim, wherein the therapeutic dose (90) is **characterized by** at least one parameter of the first light source (31) including radiant power, irradiance, exposure duration (*T*ₜₒₜ), fluence, and/or repetition intervals.

3. The apparatus (1) of any one of the preceding claims, wherein the first light source (31) is configured to emit a plurality of consecutive treatment light pulses (911) within an exposure duration (*T*ₜₒₜ), wherein each treatment light pulse (911) is **characterized by** an peak radiant power (*P*₀), a pulse period (1/f) and a pulse width (τ), wherein the exposure duration (*T*ₜₒₜ) corresponds to the time required to conclude the treatment of the selected area of the retina (120).

4. The apparatus (1) of claim 3, wherein the therapeutic dose (90) is **characterized by** the peak radiant power (*P*₀), the pulse width (τ) and/or the number of consecutive pulses within the exposure duration (*T*ₜₒₜ).

5. The apparatus (1) of any one of the preceding claims, wherein the change in the spectrum of the reflected light (93) is caused by the treatment of retinal cells while energizing the first light source (31) for applying the therapeutic dose (90).

6. The apparatus (1) of any one of the preceding claims, comprising a control device (5) connected to the first light source (31) and the retinal functional tracking means (32) and configured to control the at least one parameter of the first light source (31) in response to the determination of a change in the spectrum of the reflected light (93).

7. The apparatus (1) of claim 6, wherein the control device (5) is configured to control the at least one parameter of the first light source (31) while the first light source (31) emits a therapeutic dose (90) to the retina (120) and/or after the emission of a therapeutic dose (90).

8. The apparatus (1) of claim 6 or 7, wherein the control device (5) controls the parameter of the first light source (31) related to at least one treatment objective, wherein the at least one treatment objective is **characterized by** the thermal, biochemical and/or systemic and collective cellular activation of retinal cells.

9. The apparatus (1) of any one of the preceding claims, wherein the spectrum of the light emitted by the second light source (325) includes light in a wavelength between 700nm and 1400nm, more preferably between 700nm and 1000nm and most preferably between 700nm and 900nm.

10. The apparatus (1) of any one of the preceding claims, wherein the wavelength of the light emitted by the first light source (31) is light of a specific wavelength selected in a range from 785nm to 845nm, more preferably from 805nm to 840nm and most preferably from 825nm to 835nm.

11. The apparatus (1) of any one of the preceding claims, wherein the retinal functional tracking means (32) is configurated as an optical coherence tomograph or a confocal microscope.

12. Method for detecting a change in retinal cells' functional modification of an eye (100) stimulated by a treatment beam (91) of a first light source (31), comprising the steps of:
- Emitting a treatment beam (91) with a first wavelength to a selected area of a retina (120);
- Emitting a functional excitation beam (92) to the selected area of the retina (120), wherein the functional excitation beam (92) includes light within a spectrum;
- Receiving reflected light (93) by the retina (120) within the spectrum;
- Determining a change in the spectrum of the reflected light (93), in particular caused by an activation of mitochondrial respiration activities of retinal cells.

13. Method for detecting a change in retinal cells' of claim 12, further comprising the step of adapting a parameter of a therapeutic dose (90) in response to the determination of a change in the spectrum of the reflected light (93).

14. Method for detecting a change in retinal cells' of claim 12 or 13, wherein the treatment beam (91) and the functional excitation beam (92) are emitted simultaneously.

15. Method for detecting a change in retinal cells' of any one of the claims 12 to 14, wherein the step for determining a change in the spectrum of the reflected light (93) is performed while and/or after emitting a therapeutic dose (90).
